# EUROPEAN PATENT APPLICATION

(11) **EP 4 528 261 A1**
(43) Date of publication of application: **26.03.2025**
(21) Application number: 23807050.2
(22) Date of filing: 18.05.2023
(51) Int. Cl.: G01N 27/327

(54) **BIOSENSOR AND PREPARATION METHOD THEREFOR**

(30) Priority: 18.05.2022 CN 202210547593
(71) Applicant: Leadway (HK) Limited, Sheung Wan, Hong Kong (CN)
(72) Inventor: JI, Xubo, Hangzhou, Zhejiang 310030 (CN); LEI, Tianran, Hangzhou, Zhejiang 310030 (CN); WANG, Yi, Hangzhou, Zhejiang 310030 (CN); WU, Mengqun, Hangzhou, Zhejiang 310030 (CN); GUO, Tao, Hangzhou, Zhejiang 310030 (CN); ZHANG, Li, Hangzhou, Zhejiang 310030 (CN)
(74) Representative: Schiweck Weinzierl Koch Patentanwälte Partnerschaft mbB
(86) International application number: PCT/CN2023/095159
(87) International publication number: WO 2023/222100

(57) **Abstract**

The present invention also provides a biosensor and a preparation method thereof. The biosensor includes an insulating substrate, a sample injection port, a sample injection channel for entrance of a test sample, and a conductive layer disposed on the insulating substrate, engraved lines and electrodes divided by the engraved lines being distributed on the conductive layer, a reagent layer being disposed on part or all of the electrodes located in the region of the sample injection channel, and a short-circuit-proof region being disposed at the edge of the biosensor. The present invention can effectively overcome the risk of a short circuit between electrodes of the biosensor.

## Description

### Field of the Invention

The present invention relates to the field of biomedical testing, in particular to a biosensor and a method.

### Background of the Invention

Electrochemical sensors for detecting the content of an analyte in a sample and their matched detectors have been widely used in the daily monitoring of diseases. For example, diabetic patients commonly use electrochemical sensors to monitor daily glucose concentrations in the blood.

The basic structure of such electrochemical sensors includes an electrode system disposed on an insulating substrate, the electrode system includes a plurality of or multiple types of electrodes such as a working electrode and a counter electrode, and a reaction reagent that reacts with the analyte covers the corresponding electrodes. A sample interlayer having a groove is located on the electrodes, a cover plate with an air hole covers the sample interlayer, the insulating substrate, the interlayer and the cover plate form a sample injection channel, and the other end of the electrode system contacts a contact of a detector. The sample flowing into the sample injection channel reacts with a reaction reagent on the electrodes to generate electrical signals from which the detector derives the test result.

The screen printing technology is utilized to print conductive materials on the insulating substrate to form electrodes, electrode connecting contacts and leads, which is a method commonly used for preparing electrochemical sensors at present. When electrochemical sensors are manufactured using the screen printing method, large batch-to-batch variations exist in aspects of certain parameters from batch to batch. In order to eliminate the extent to which the batch-to-batch variations affect the test results, the problem of batch-to-batch variations is generally solved by inserting a calibration chip into the detector to correct the test results. However, inserting a calibration chip not only increases the operation steps of an operator, but also leads to inaccurate test results if the operator forgets to insert or incorrectly inserts the calibration chip. Some researchers also adopted, for example, the method in Chinese Patent ZL201210095096.5 to correct the batch-to-batch variations by setting calibration messages on the sensor.

In order to solve the problem of cumbersome testing steps or complicated preparation process mentioned above, Chinese Patent ZL00803756.6 provides a method for forming a thin film electrode, i.e., a thin film-like conductor layer is uniformly spread on an insulating substrate in advance, and then the electrode is formed by a laser etching method. This method has relatively high manufacturing precision and basically can ensure no batch-to-batch variations between products produced in different batches, or that the presence of batch-to-batch variations will not affect the test results in the case where no correction is required.

The process of uniformly spreading the thin film-like conductive layer on the insulating substrate determines the adhesion of the conductive layer to the insulating substrate, and different processes, different conductive layers, and insulating substrates made of different materials are all factors affecting the adhesion. Whether the adhesion of the conductive layer to the insulating substrate is good or bad directly affects the cutting performance of the sensor. In the process of using a cutter to cut semi-finished film sensors into single independent finished sensors, metal conductive layers such as gold and palladium, or non-metallic conductive layers such as carbon and conductive glass will generate metallic conductive particles or non-metallic conductive particles due to differences of the adhesion and the mechanical shear force of the cutting process to the conductive layer, and the particles may remain in the engraved line close to the travel line of the cutter, so that a plurality of electrode regions that should have been disconnected from each other are connected to form a short-circuit situation, and the obtained finished sensors can only be scrapped.

The biosensor contains an insulating substrate and a conductive layer disposed on the insulating substrate, engraved lines being distributed on the conductive layer, and electrodes being formed on the conductive layer and divided by the engraved lines. A test reagent is added to all or part of the electrodes at the sample contacting end. The biosensor further includes a fluid channel for entrance of the solution of a substance to be tested, and the fluid channel is formed by the conductive layer, an open groove of an interlayer, and an upper cover together. The electrodes extend from the sample contacting end to the contact end of the sensor and conduct an electrical signal generated by the reaction of the substance to be tested with the reagent to the contact end of the sensor, and then the test result is fed back to the user via an instrument. After the cutting process of the sensor is completed, a quality inspector will test the sensor to determine whether the obtained sensor is qualified, in which it is a particularly important step that whether the sensor is scrapped due to a short circuit occurring between the electrodes resulted from the cutting.

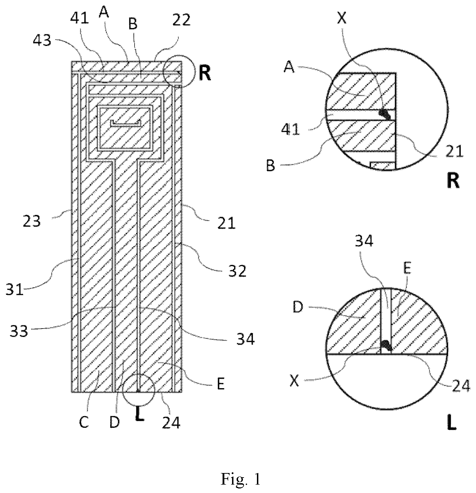

Specifically, in the process of cutting semi-finished biosensors into independent finished biosensors, mechanical shear to the conductive layer exists on the travel line of the cutter, the conductive layer after being stressed has a certain possibility to result in peeling off or detachment of the conductive particles, so as to remain in nearby engraved lines. Fig. 1 shows a schematic diagram of the conductive layer of a thin-film electrode biosensor, wherein 21, 22, 23, 24 are edge lines of the conductive layer obtained after the cutter cuts along a preset line, and the travel line of the cutter intersects engraved lines 31, 32, 33, 34, 41, 43. L of Fig. 1 and R of Fig 1 are enlarged views of regions selected at the instrument contact end and the sample contacting end of the illustrated sensor, respectively, and electrodes D and E on the left and right sides of the engraved line 34 are divided by the engraved line 34, and the electrode D and the electrode E should be disconnected from each other and have no electrical conduction between them. When the cutter cuts along the preset line to obtain the edge line 24 of the biosensor, and the cutter travels to the engraved line 34, as the conductive layer is subjected to a certain mechanical shear force, there may be conductive particles X peeling off or detaching to remain in the engraved line 34, thereby resulting in that the electrodes D and E are electrically conductive through the conductive particles X, that is, the electrodes D and E are short-circuited. When the sensor to which this phenomenon occurs is inserted into a test instrument, the test instrument will automatically alarm when it is found through testing that a short-circuit situation exists between the electrodes, and it cannot be used by a user.

Similarly, when the cutter cuts along the preset line to obtain a biosensor edge line 21, and the cutter travels to the engraved line 41, it may also happen that the peeled conductive particles X will remain in the engraved line 41, thereby resulting in electrical conduction between electrode B and a non-electrode conductive block domain A other than the electrode B. The non-electrode conductive block domain A does not participate in the conduction of electrochemical signals, and the electrode B is connected to electrode C at the instrument contact end, and the two electrodes jointly form an electrode participating in the conduction of electrochemical signals. As a result of the short circuit occurring between the non-electrode conductive block domain A and the electrode B, the size of the electrode B of the sensor changes, i.e., the electrode size of the electrode B also encompasses the non-electrode conductive block domain A with which the electrode B is short-circuited, which may lead to fluctuations of conduction signals to cause deviations of sensor test values. In the figure indication of Fig. 1, the electrodes B and C point to the same electrode, and the electrode B and the electrode C are the same electrode.

Sensors, of which the electrodes that should have been disconnected from each other are short-circuited as conductive particles remain in the engraved line, or sensors, of which the electrode sizes fluctuate as conductive particles remain in the common engraved line, are defective scrapped products that cannot be used by customers. Due to the small size of the conductive particles, it is not possible to quickly determine with naked eyes.

### Summary of the Invention

The present invention is completed based on the above problems in the prior art, and has an objective of providing a biosensor which can avoid the risk of a short circuit occurring between electrodes or between electrodes or non-electrode conductive block domains other than the electrodes.

The short-circuit-proof region consists of the engraved lines and the edge lines of the biosensor jointly, with the main objective of avoiding the risk of sensor scrapping resulted from electrical conduction occurring between electrodes or between electrodes and non-electrode conductive block domains other than the electrodes. The short-circuit-proof region is arranged at the peripheral edge of the sensor or is relatively close to the edge line of the biosensor.

As the laser etching process has a large customization space, a skilled person can design the travel lines of the engraved lines according to the actual situation, or carry out customized setting on etching parameters of the engraved lines, so the design solution of the short-circuit-proof region has a variety of forms. Specifically, the short-circuit-proof region can be enclosed by a plurality of independent engraved lines and the edge lines of the conductive layers. All of the above solutions can be well realized to avoid the risk that the non-electrode conductive block domains on both sides of engraved lines of the biosensor are connected by the residual conductive particles, and have strong operability and practicality. The above solution will be stated specifically below in conjunction with specific embodiments.

Therefore, the present invention provides a biosensor, comprising an insulating substrate, a sample injection port, a sample injection channel for entrance of a test sample, and a conductive layer disposed on the insulating substrate, engraved lines and electrodes divided by the engraved lines being distributed on the conductive layer, a reagent layer being disposed on part or all of the electrodes located in the region of the sample injection channel, wherein on the path that two adjacent electrodes extend towards the edge of the biosensor, at least one electrode does not extend to the edge of the biosensor; or on the path that an electrode and a non-electrode conductive block adjacent thereto extend towards the edge of the biosensor, at least one of the electrode and the non-electrode conductive block does not extend to the edge of the biosensor. Further, a non-electrode conductive block is disposed between at least one of two adjacent electrodes and the edge of the biosensor.

Further, a short-circuit-proof region is disposed between the electrode not extending to the edge of the biosensor or the conductive block not extending to the edge of the biosensor and the edge of the biosensor.

Further, the short-circuit-proof region is enclosed by the edge lines of the conductive layer and the engraved lines.

Further, none of the adjacent electrodes extends to the edge of the biosensor, or neither of the adjacent electrodes nor the non-electrode conductive block extends to the edge of the biosensor.

Further, the engraved lines on the conductive layer do not intersect edge lines the biosensor.

Further, the conductive layer is selected from gold, silver, platinum, palladium, carbon, graphite, conductive glass, or a mixture thereof.

The present invention also provides method for preparing a biosensor, comprising the following steps: on an original material having an insulating substrate and a conductive layer, etching engraved lines on the conductive layer and forming electrodes divided by the engraved lines; adding a prepared reagent to corresponding electrodes; and sticking an interlayer and an upper cover sequentially on the electrodes to form the biosensor with a sample injection channel; on the path that two adjacent electrodes extend towards the edge of the biosensor, at least one electrode does not extend to the edge of the biosensor; or on the path that an electrode and a non-electrode conductive block adjacent thereto extend towards the edge of the biosensor, at least one of the electrode and the non-electrode conductive block does not extend to the edge of the biosensor.

Further, the conductive layer is selected from gold, silver, platinum, palladium, carbon, graphite, conductive glass, or a mixture thereof.

Further, the etching method is laser cutting or laser marking.

The present invention also provides a method of avoiding a short circuit of electrodes of a biosensor, wherein on the path that two adjacent electrodes extend towards the edge of the biosensor, at least one electrode does not extend to the edge of the biosensor; or on the path that an electrode and a non-electrode conductive block adjacent thereto extend towards the edge of the biosensor, at least one of the electrode and the non-electrode conductive block does not extend to the edge of the biosensor; and a non-electrode conductive block is disposed between at least one of two adjacent electrodes and the edge of the biosensor.

The present invention proposes a solution of setting up the short-circuit-proof region on the conductive layer through the controllable design of the engraved lines, which can effectively avoid the risk that the non-electrode conductive block domains on both sides of the engraved lines are connected by the residual conductive particles, in order to ensure the stable performance of the finished sensors and to improve the finished product yield of the sensors.

### Brief Description of the Drawings

Fig. 1 is a schematic diagram of the distribution of electrodes and engraved lines on a conductive layer of an electrode biosensor having no short-circuit-proof region.
Fig. 2 is an exploded schematic diagram of a biosensor of the present invention.
Fig. 3 is a flow chart of a manufacturing method of the biosensor.
Fig. 4 is a schematic diagram of multi-lined sensor base units A formed by laser engraving of the conductive layer of the biosensor.
Fig. 5 is a schematic diagram of the single-lined sensor base units A formed after cutting by a cutter along the cutting line 50 as shown in Fig. 4.
Fig. 6 is a schematic diagram of the design of a short-circuit-proof region in Example 1, wherein R and L are enlarged views of the left figure.
Fig. 6-1 is a schematic diagram of the design of another two short-circuit-proof regions of Example 1.
Fig. 6-2 is a schematic diagram of another short-circuit-proof design, wherein R and L are enlarged views of the left figure.
Fig. 7 is a line graph of glucose sensor test values versus YSI test values of Example 3.

### Detailed Description of the Embodiments

Embodiments of the present invention will be described below with reference to the drawings, but the present invention is not limited to the listed specific examples.

### Example 1:

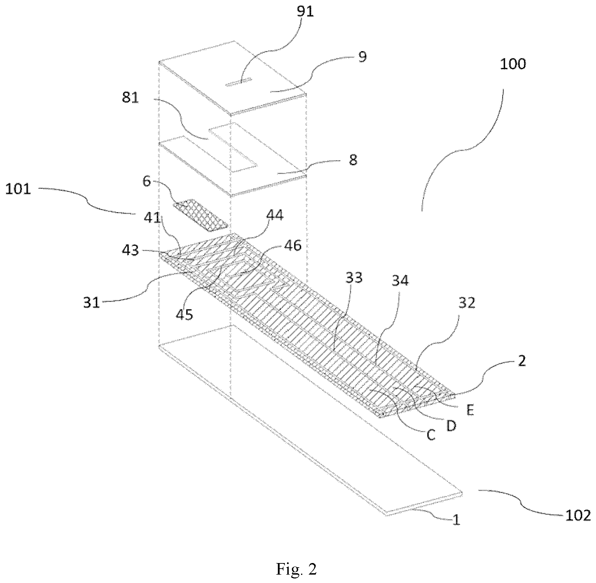

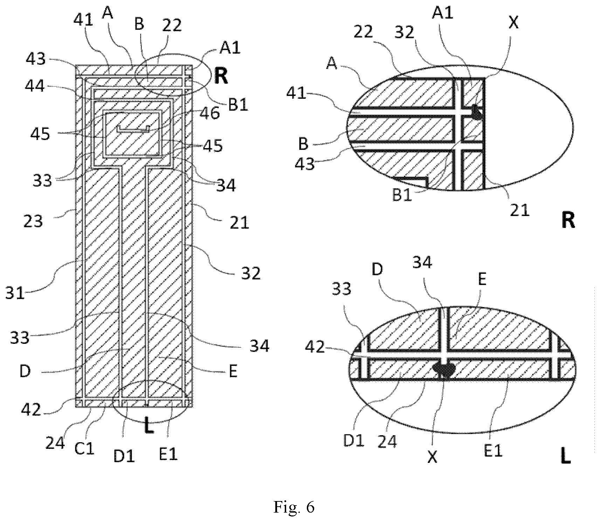

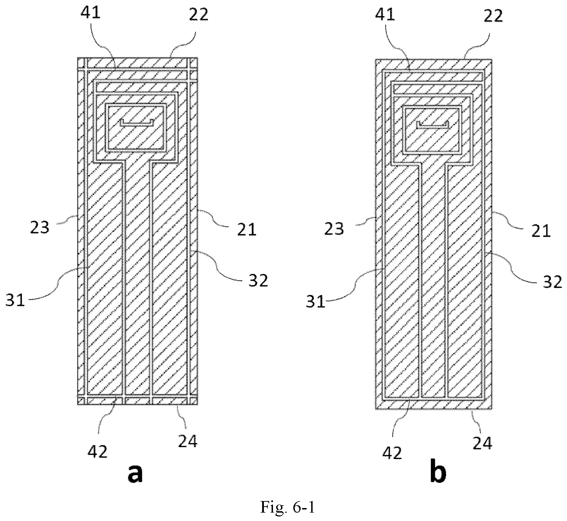

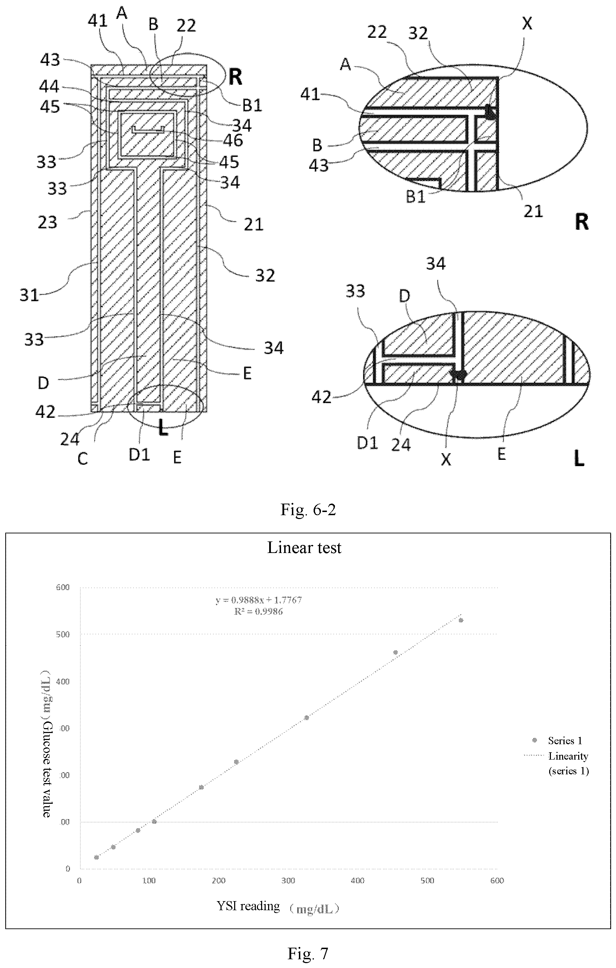

A biosensor 100 as shown in Figs. 2 and 6 includes an insulating substrate 1, and an electrode system disposed on the substrate and formed through dividing a conductive layer 2 via engraved lines. The electrode system includes a working electrode E, a counter electrode D and a reference electrode C, and the electrodes have a sample contacting end 101 contacting a sample and a contact end 102 contacting a contact of an analyzer. A reagent layer 6 is added to corresponding electrodes at the sample contacting end. An interlayer 8 with an open groove 81 covers the sample contacting end of the electrode, and an upper cover 9 covers the interlayer 8, so that the conductive layer 2, the open groove 81 and the upper cover 9 form a sample injection channel. The electrodes and the reagent layer are in the sample injection channel. The upper cover 9 is provided with an air hole 91, which is located above the open groove and used for discharging air in the sample injection channel after sample addition.

Engraved lines and independent conductive blocks divided by the engraved lines are distributed on the conductive layer 2. These independent conductive blocks may be divided into electrodes and non-electrode conductive blocks. The electrode refers to a conductive block, a part of which is located in the sample injection channel, and the rest part of which extends towards the contact end of the biosensor, for example, electrodes C, D, E. The non-electrode conductive block refers to a conductive block other than the electrodes, for example, conductive blocks A, A1, B1, C1, D1, E1, and a region enclosed by the engraved lines 41, 31, 42 and the edge lines. In the figure indication of Figs. 2, 6-1 and 6-2, the electrodes B and C point to the same electrode, and the electrode B and the electrode C are the same electrode.

The electrodes are formed after dividing the conductive layer 2 by the engraved lines, the engraved lines are insulating gaps left by the removal of a conductive material, and the engraved lines are not limited to gaps formed by means of engraving. The front view of the conductive layer 2 corresponding to Fig. 2 is as shown in Fig. 6. The working electrode E is enclosed by the engraved lines 32, 33, 34, 42, 43, 44, the counter electrode D is enclosed by the engraved lines 33, 34, 42, 44, 45, and the reference electrode C is enclosed by the engraved lines 31, 32, 33, 41, 42, 43.

The engraved lines described in the present invention are not only straight lines, but also can be arcs, wavy lines and other curves. The transverse engraved line does not mean that it needs to be parallel to the two end edges of the sample end and contact end of the biosensor, and the transverse engraved line may be parallel to the end edges or at an angle to the end edges. The longitudinal engraved line also does not mean that it needs to be parallel to two side edges of the biosensor, and the longitudinal engraved line may be parallel to the side edges or at an angle to the side edges.

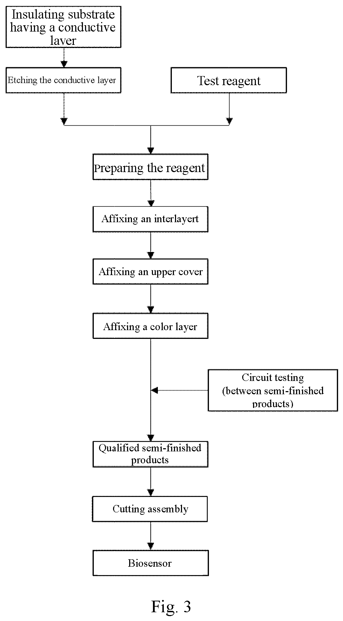

A method for manufacturing the biosensor shown in Fig. 2 is illustrated by taking Figs. 3, 4 and 5 as examples.

Step 1: laser etching of an electrode pattern: according to a pre-designed electrode pattern, on an original material having an insulating substrate and a conductive layer, etching the electrode pattern with laser on the conductive layer, thus obtaining a large electrode card with a plurality of sensor base units A.

Step 2: addition of the reagent layer: preparing a test reagent, and adding the prepared reagent to corresponding electrodes requiring addition of the reagent.

Step 3: affixing the interlayer 8 to each sensor base unit A. Generally, the interlayer is placed at the sample contacting end of the biosensor.

Step 4: sticking the upper cover 9 to each interlayer 8 and rolling.

Step 5: after the upper cover is affixed, affixing and rolling a color layer on the upper cover to obtain a semi-finished large card.

Step 6: cutting: cutting the semi-finished large card by using a cutter along a preset cutting line to obtain finished biosensors.

Specifically, in step 1, the conductive layer 2 on the surface of the insulating substrate is etched using the laser etching technology to form a plurality of engraved lines 31 to 34 and engraved lines 41 to 46 on the conductive layer 2, and the conductive layer in the engraved lines is removed to expose the insulating substrate. Through division by the engraved lines, the working electrode E, the counter electrode D, the reference electrode C are formed on the conductive layer. After laser etching is completed, a large electrode card is obtained, including a plurality of sensor base units A on an insulating substrate, which are arranged side by side and can be used to manufacture finished biosensors. Each sensor base unit A includes an insulating substrate, an electrode system formed after division by engraved lines, the engraved lines, and other blocks formed by etching. In this example, the electrode system includes the working electrode E, the counter electrode D, the reference electrode C, and a short-circuit-proof region.

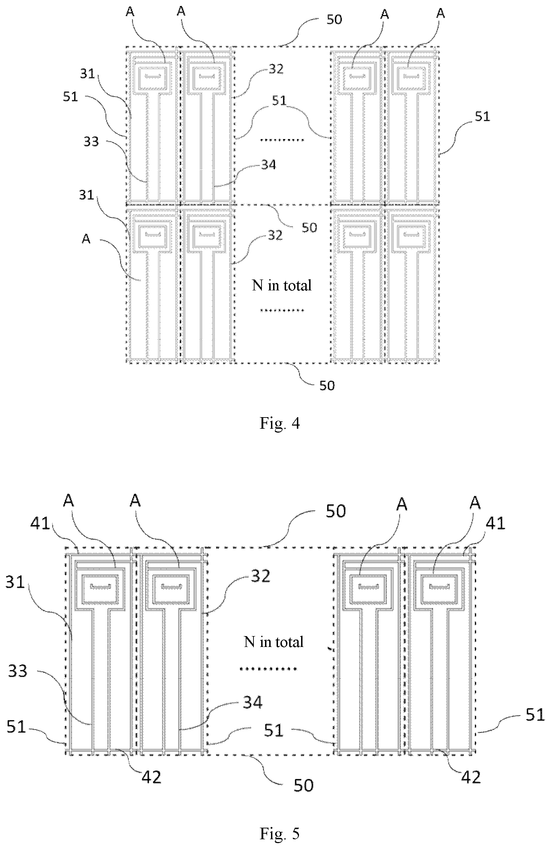

As shown in Fig. 4, two rows of sensor base units A arranged in sequence end to end are formed after laser etching (the contact ends of the two sensor base units A are opposed to each other up and down), wherein N represents 0 to N; and "N in total" represents inclusion of N sensor base units A.

In step 1, i.e., laser etching of an electrode pattern, the etching travel line of the engraved lines within the conductive layer is customized by software. The specific implementation of the path and direction and the like of the engraved lines can be artificially designed and controlled by software according to the needs of actual products, and is not limited to only one path. Similarly, the software also can apply different laser etching parameters to the engraved lines, the width of the available engraved lines is within the range of 0.020mm to 0.200mm, and the width of the engraved lines preferred in this example is 0.080mm. Based on the basic performance of laser etching equipment, some of the equipment and software can also achieve a complete stripping mode of patterns, and the specific embodiments will be introduced subsequently.

The material of the insulating substrate is polyvinyl chloride, polyterephthalic acid, polyethylene terephthalate, macrogol ester, etc., with polyethylene terephthalate being preferred in this example.

The material of the conductive layer 2 may be gold, silver, platinum, palladium, carbon, graphite, conductive glass, and other conductive metal or conductive non-metal not limited thereto, or a mixture thereof. The method for covering the conductive layer may use printing, coating, electroplating, sputtering, etc., and this example uses a conductive carbon layer (carbon film) formed in a coating manner, the thickness of the carbon layer is 1-30um, the thickness used in this example is about 8um, and the resistance of the conductive layer is 10 Ω/□ to 100 Ω/□, preferably 30 Ω/□ in this example.

Some or all of the electrodes of the biosensor may be configured with a reagent layer. The reagent layer employs a specific enzyme to quantitatively or qualitatively analyze the measured target substance under the environment of a buffer system, generally containing the following components: enzyme, electronic mediator, high polymer, disintegrant, surfactant, stabilizer and buffer system. Depending on the needs of testing items, the enzyme is selected from one or more of glucose oxidase, glucose dehydrogenase, lactate oxidase, lactate dehydrogenase, urate oxidase, cholesterol oxidase or D-3-hydroxybutyrate oxidase, etc.; the electronic mediator is selected from one or more of ruthenium compounds, potassium ferricyanide, ferrocene, etc.; the buffer system is any one or more of sodium succinate, sodium citrate, piperazine buffer, propanesulfonic acid, PBS buffer or sodium fumarate; the disintegrant is any one or more of crosslinked PVP, sodium carboxymethyl starch, or crosslinked CCNa; the surfactant is any one or more of anionic surfactant, cationic surfactant, diatomic surfactant, or nonionic surfactant; and the stabilizer is one or more of maltitol, trehalose, BSA, or protein protective agent. The above components after preparation are stirred sufficiently, so that the components are fully dissolved and dispersed to form a homogeneous solution. In this example, the biosensor as shown in Fig. 1 includes a reagent layer 6.

In step 2 for preparing the reaction reagent, methods such as dispensing, screen printing, drop coating and Slot-Die coating are usually adopted to configure a reagent mixture having certain chemical components onto a specific electrode of the biosensor to form the reagent layer. It is preferred in this example that screen printing is used to configure a reagent on the specific electrode in specific pattern and position. The biochemical reagent in the specific pattern and position can react with the substance to be tested in the sample and generate a certain electrical signal, and the electrical signal is conducted to a test instrument via a conductive material and fed back to a user. The screen printing plate used in the screen printing generally employs polyester, nylon, stainless steel and other materials, and is generally of 250 meshes to 420 meshes, the used wire screen has a wire diameter of 27um to 120um generally, and bears a maximum tension of 22 to 38N/cm generally. It is preferred in this example that a 305-mesh nylon screen printing plate with a wire diameter of 34um is used, which can bear a maximum tension of 33N/cm.

The above biochemical reagent with fixed pattern and position obtained via screen printing comes into contact with the sample in the sample injection channel and reacts with the substance to be tested in the sample. The sample injection channel is formed between the open groove 81 and the upper cover 9, and the surface of the upper cover facing the sample injection channel is a hydrophilic layer material. Specifically, the material of the interlayer is typically a PET substrate coated with an acrylic resin system as an adhesive material. The thickness of the interlayer is typically 75um to 150um, and the width of the open groove is typically 0.7mm to 1.8mm. In this example, the thickness of the interlayer is preferably 100um, and the width of the hollow structure is preferably 1.2mm.

An air hole 91 is left in the upper cover 9 at the tail of the sample injection channel, and the air hole may be round, square, rectangular, linear, or in other shapes. It is preferred in this example that the air hole is rectangular. In this example, the hydrophilic material is preferably 9901P made by 3M. The air hole of the hydrophilic material can ensure that the original air in a chamber is smoothly discharged when a blood sample flows into the sample injection channel, so as to ensure that the sample can smoothly flow into the chamber.

The color layer is generally a printable single-sided adhesive tape, with the product name generally printed on the surface, which plays a role of easily identifying the product and protecting the biosensor from damage caused by scratching.

Step 5 is a step that can be omitted. For example, if the upper cover itself can be printed with product name, which can simultaneously serve a similar function as the color layer, step 5 is omitted. Alternatively, if the biosensor does not require the color layer, step 5 is omitted.

In the production process, the material which has the color layer of the upper cover of the interlayer being affixed and has a plurality of sensor base units A is referred to as a semi-finished large card, or the material completing steps 1 to 4 or steps 1 to 5 is referred to as a semi-finished large card.

In the cutting step 6, the semi-finished large card is cut to obtain finished biosensors by using a cutter along a preset cutting line, specifically, using the cutter along the cutting lines at positions shown by transverse dotted lines 50 and longitudinal dotted lines 51 in Fig. 4. Suitable cutting methods include but not limited to hobbing, die punching, chopping, etc. Specifically, it is preferred in this example to use a hob to hob the semi-finished large card as shown in Fig. 4 along the transverse dotted line 50 into semi-finished strip-shaped sheets containing several sensor base units A as shown in Fig. 5, and then use the hob to cut the semi-finished strip-shaped sheets along the vertical dotted line 51 to obtain finished biosensors. In order to more visually illustrate the cutting position of the hob, when Figs. 4 and 5 are used to introduce the cutting process of step 6, the interlayer and the upper cover are not shown in Figs. 4 and 5. The cutting process described in step 6 is one of the important steps in the assembly of the sensor, the effect of which directly affects the pass rate of the final finished sensors and the finished product effect of the sensors. The dotted lines labeled in Fig. 4 and Fig. 5 do not exist in the actual product processing, the same below. After cutting, the positions of the dotted lines 50 and 51 are edge lines of the biosensor, or can also be referred to as edges of the biosensor or side edges of the biosensor.

In the cutting process of a full-layout semi-finished product, in order to ensure the finished product pass rate of the products, after the hob cuts the semi-finished large card along the preset horizontal dotted line 50 into strip-shaped sheets, the quality inspector of the production process first checks whether the engraved lines 41, 42 in the upper sample channel are complete, and if they are complete, then it can be determined that the strip-shaped sheets obtained via cutting are semi-finished products of qualified products. When the hob cuts the strip-shaped sheets along the preset vertical dotted line 51, the quality inspector of the production process checks again whether the cutting position is located in the gap between two sensor units, i.e., if the engraved lines 31 and 32 on two sides can be seen completely, the finished biosensor is a qualified product biosensor. If the engraved lines 31, 32, 41 and 42 of the biosensor obtained after cutting are complete engraved lines, it indicates that the biosensor obtained from the manufacturing is a qualified product. Therefore, by utilizing the engraved lines 31, 32, 41 and 42 as quality control lines, the completeness of the engraved lines 31, 32, 41 and 42 is subjected to quality inspection during the production process, and the defective products not qualifiedly cut can be effectively removed.

However, as the conductive particles remaining in the engraved line, resulted from the mechanical shear force of the cutter on the conductive layer, are difficult to be identified by the quality inspector with naked eyes, the generated risk is mainly that it may cause a short circuit of adjacent electrodes or electrical conduction of the electrodes and the non-electrode conductive block domains other than the electrodes, and the obtained sensor is also an unqualified product, which cannot be circulated into the next production process. In order to eliminate the risk of generating such unqualified sensors, the present invention proposes a design solution for forming a short-circuit-proof region using the engraved lines and the edge lines of the conductive layer. It will be specifically stated below in conjunction with Fig. 6. Compared with at the contact end of the biosensor in Fig. 1, the biosensor shown in Fig. 6 is provided with an engraved line 42 at the contact end, non-electrode conductive blocks C1, D1, E1 are enclosed by the engraved line 42 and the engraved lines 31, 32, 33, 34 together with the edge line 24 of the biosensor, and more specifically, the non-electrode conductive block C1 is enclosed by the engraved lines 42, 31, 33 and the edge line 24 of the biosensor; the non-electrode conductive block D1 is enclosed by the engraved lines 42, 33, 34 and the edge line 24 of the biosensor; and the non-electrode conductive block E1 is enclosed by the engraved lines 42, 34, 32 and the edge line 24 of the biosensor. The non-electrode conductive blocks C1, D1, E1 located at the contact end of the sensor are directly adjacent to the edge line 24 of the biosensor and are independent to each other. Taking the non-electrode conductive blocks D1 and E1 as examples, as in the enlarged view of the selected region L at the contact end of the sensor in Fig. 6, X is conductive particles X remaining in the engraved line 34 when the cutter cuts along the preset line to obtain the end edge 24 of the sensor. The conductive particles X connect the non-electrode conductive blocks D1 and E1 which should have been spaced by the engraved line 34 and not connected, resulting in a short circuit of D1 and E1. Due to the presence of the engraved line 42, the non-electrode conductive block D1 and the electrode D, as well as the non-electrode conductive block E1 and the electrode E are spaced by the engraved line 42 and not connected. That is to say, even if D1 and E1 are short-circuited, the electrodes D and E remain in a state of being disconnected from each other and are not affected by the fact that the conductive particles X remain in the engraved line 34. The conductive blocks D1, E1 are short-circuit-proof regions at the contact end of the sensor, which can avoid electrical conduction between the electrode D and the electrode E as the conductive particles X fall into the engraved line 34.

Similarly, as shown in Fig. 6, in comparison to Fig. 1, the engraved line 32 extends to the edge line 22 of the biosensor at the sample contacting end of the sensor. A non-electrode conductive block B1 is enclosed by the engraved line 41 together with the engraved lines 32, 43 and the edge line 21 of the biosensor, and a non-electrode conductive block A1 is enclosed by the engraved line 41 together with the engraved line 32 and the edge lines 21, 22 of the biosensor. The non-electrode conductive blocks A1 and B1 located at the sample contacting end of the sensor are directly adjacent to the edge line 21 of the biosensor and are independent of each other, and as in the enlarged view of the selected region R at the sample contacting end of the sensor in Fig. 6, X is conductive particles X remaining in the engraved line 41 when the cutter cuts along the preset line to obtain the end edge 21 of the sensor. The conductive particles electrically conduct the conductive blocks A1 and B1, which should have been spaced by the engraved line 41 and not connected, resulting in a short circuit of A1 and B1. Due to the presence of the engraved line 32, the non-electrode conductive block A1 and the non-electrode conductive block A, as well as the non-electrode conductive block B1 and the electrode B are spaced by the engraved line 32 and not connected. That is to say, even if A1 and B1 are short-circuited, the non-electrode conductive block A and the electrode B remain in the state of being disconnected from each other and are not affected by the fact that the conductive particles X remain in the engraved line 41. The non-electrode conductive blocks A1, B1 are short-circuit-proof regions at the sample contacting end, which can avoid electrical conduction between the electrode B and the conductive block A, and thus avoids the change of the area of the electrode B due to the fact that the conductive particles X remain in the engraved line.

Through the above design of the short-circuit-proof regions formed by the edge lines of the biosensor together with the engraved lines, electrical conduction between the electrodes or between electrodes and the conductive layer other than the electrodes can be effectively avoided, which greatly improves the accuracy and finished product pass rate of the product.

Fig. 6-1 is an improved design on the basis of Fig. 6. As shown in a of Fig. 6-1, the non-electrode conductive block domain around the sensor, which is enclosed by the engraved lines 41, 42, 31, 32 together with the edge lines 21, 22, 23, 24 of the biosensor, can also be considered as a form of the short-circuit-proof region, which is functionally consistent with the preceding instance. As shown in b of Fig. 6-1, all of the engraved lines that may extend to the edge line of the biosensor terminate only at the engraved lines 41, 42, 31, 32, and the non-electrode conductive block domain around the sensor, enclosed by the engraved lines 41, 42, 31, 32 together with the biosensor edge lines 21, 22, 23, 24, is also still another form of the short-circuit-proof region, and can still be used to avoid electrical conduction between electrodes or between electrodes and the conductive layer other than the electrodes due to the electrically conductive particles generated by cutting.

The biosensor as shown in Fig. 6-2 includes electrodes C, D, E and non-electrode conductive blocks B1, D1, etc. In the design solution of Fig. 6-2, the electrode E is adjacent to the electrode D and the non-electrode conductive block D1, wherein the non-electrode conductive block D1 is disposed between the electrode D and the edge 24 of the biosensor. As shown in the enlarged view at L of Fig. 6-2, after cutting with the cutter, even if the conductive particles X fall on the engraved line 34 close to the edge of the sensor, it will not result in the occurrence of a short circuit between the electrode E and the electrode D.

### Example 2: glucose test data

The present invention will be further illustrated below in conjunction with the drawings and Figs. 2 and 6 of the solution in Example 1.

As shown in Fig.2, the glucose biosensor in this example includes an insulating substrate 1, a conductive carbon layer 2, a reagent layer 6, an interlayer 8 and an upper cover 9. Specifically, the glucose biosensor of this example includes a conductive carbon coating 2 of uniform thickness, which is prepared by a coating process and located on the insulating substrate 1. Laser engraved lines 31, 32, 33, 34, 41, 42, 43, 44, 45, 46 and the like are etched on the conductive carbon coating using the laser etching technology according to a reasonable programming design, and the conductive layer is removed by ablation of the laser engraved lines to expose the insulating substrate, thus forming electrodes C, D, E on the conductive layer 2 that are not connected to each other. The reagent layer 6 is located on the electrodes and in a sample injection groove 81 of the interlayer 8. The interlayer 8 is covered with the upper cover 9, and the air hole 91 in the upper cover is located at the bottom of the sample injection groove 81 of the interlayer. The sample injection groove 81 of the interlayer, and the hydrophilic upper cover 9 and the air hole 91 above the sample injection groove form a blood sample injection channel.

After the glucose sensor is inserted into the test instrument, the test instrument is started, the blood sample is sucked into the sample injection channel by siphoning, and the air in the original sample injection channel is discharged through the air hole 91 in the upper cover at the tail end of the sample injection channel, which effectively ensures the smoothness that the blood flows into the channel. When the blood enters to fill in the channel, a DC voltage of 200-500mv is applied to the electrode so that the reagent layer 6 carries out a redox reaction with the glucose to be tested in the blood and generates a test current. The test current is appropriately corrected and compensated for by the test instrument according to the detected current value and the ambient temperature measured by a temperature sensor, and is converted into a glucose value for display to the user.

The materials of the reagent layer 6 mainly include glucose dehydrogenase FAD-GDH, potassium ferricyanide and a second electron enzyme mediator; and the enzyme activity of the glucose dehydrogenase is within the range of 200-600U/mg.

11 blood samples with different glucose concentrations are tested at room temperature using the above glucose sensor. The hematocrits of different concentrations of blood samples are adjusted to 42% ± 2% before the test, and their specific concentrations are obtained through testing with a glucose lactate analyzer (model YSI 2300 STAT) manufactured by YSI Corporation, U.S.A. The test is repeated 10 times for each concentration of blood sample, and the test results are shown in Table 1.

**Table 1 Test results of glucose sensor**

| YSI reading (mg/dl) | 11 | 23 | 47 | 83 | 106 | 174 | 224 | 325 | 453 | 547 | 647 |
|---|---|---|---|---|---|---|---|---|---|---|---|
| Reading 1 | LO | 25 | 48 | 83 | 104 | 175 | 230 | 329 | 461 | 520 | HI |
| Reading 2 | LO | 26 | 46 | 84 | 100 | 170 | 229 | 323 | 452 | 518 | HI |
| Reading 3 | LO | 26 | 47 | 82 | 108 | 181 | 238 | 317 | 455 | 529 | HI |
| Reading 4 | LO | 25 | 47 | 82 | 104 | 172 | 228 | 324 | 461 | 522 | HI |
| Reading 5 | LO | 24 | 46 | 84 | 102 | 175 | 229 | 327 | 459 | 554 | HI |
| Reading 6 | LO | 24 | 47 | 83 | 101 | 175 | 221 | 317 | 459 | 520 | HI |
| Reading 7 | LO | 25 | 47 | 81 | 97 | 178 | 235 | 323 | 459 | 540 | HI |
| Reading 8 | LO | 26 | 48 | 83 | 99 | 172 | 235 | 328 | 476 | 561 | HI |
| Reading 9 | LO | 23 | 46 | 81 | 99 | 174 | 221 | 319 | 474 | 515 | HI |
| Reading 10 | LO | 25 | 46 | 81 | 100 | 176 | 225 | 326 | 469 | 522 | HI |
| Average value | ---- | 24.9 | 46.8 | 82.4 | 101.4 | 174.8 | 229.1 | 232.3 | 462.5 | 530.1 | ---- |
| Standard deviation | ---- | 0.99 | 0.79 | 1.17 | 3.20 | 3.16 | 5.76 | 4.40 | 7.92 | 16.11 | ---- |
| Test precision | ---- | 4.0% | 1.7% | 1.4% | 3.2% | 1.8% | 2.5% | 1.4% | 1.7% | 3.0% | ---- |
| Test deviation | ---- | 2.0 | -0.6 | -0.2 | -4.2% | 0.4% | 2.2% | -0.6% | 2.2% | -3.0% | ---- |

It can be seen from the comparison of the average of 10 repeated tests of blood at each glucose concentration and the test results of YSI 2300 that in the range of very low glucose concentration (less than 20mg/dl) and in the range of very high glucose concentration (more than 600mg/dl), the test results of the instrument show LO and HI, respectively. Except for at these extreme concentrations, the glucose sensor has very small test deviation and high accuracy. In addition, the coefficients of variation of the test values of the same blood sample are all within 3%, and the precision completely meets requirements.

Fig. 7 is a line graph of the glucose sensor test values versus the YSI test values of Example 2, with a fitting equation y = 0.9888x + 1.7767; and R² = 0.9986. It can be seen that the glucose sensor has a good line shape.

## Claims

1. A biosensor, comprising an insulating substrate, a sample injection port, a sample injection channel for entrance of a test sample, and a conductive layer disposed on the insulating substrate, engraved lines and electrodes divided by the engraved lines being distributed on the conductive layer, a reagent layer being disposed on part or all of the electrodes located in the region of the sample injection channel, wherein on the path that two adjacent electrodes extend towards the edge of the biosensor, at least one electrode does not extend to the edge of the biosensor; or on the path that an electrode and a non-electrode conductive block adjacent thereto extend towards the edge of the biosensor, at least one of the electrode and the non-electrode conductive block does
not extend to the edge of the biosensor.

2. The biosensor according to claim 1, wherein a non-electrode conductive block is disposed between at least one of two adjacent electrodes and the edge of the sensor.

3. The biosensor according to claim 1, wherein a short-circuit-proof region is disposed between the electrode not extending to the edge of the biosensor or the conductive block not extending to the edge of the biosensor and the edge of the biosensor.

4. The biosensor according to claim 1, wherein the short-circuit-proof region is enclosed by the edge of the biosensor and the engraved lines.

5. The biosensor according to claim 1, wherein none of the adjacent electrodes extends to the edge of the biosensor, or neither of the adjacent electrodes nor the non-electrode conductive block extends to the edge of the biosensor.

6. The biosensor according to claim 1, wherein the engraved lines on the conductive layer do not intersect the edge lines of the biosensor.

7. The biosensor according to claim 1, wherein the conductive layer is selected from gold, silver, platinum, palladium, carbon, graphite, conductive glass, or a mixture thereof.

8. A method for preparing a biosensor, comprising the following steps: on an original material having an insulating substrate and a conductive layer, etching engraved lines on the conductive layer and forming electrodes divided by the engraved lines; adding a prepared reagent to corresponding electrodes; and sticking an interlayer and an upper cover sequentially on the electrodes to form the biosensor with a sample injection channel, wherein on the path that two adjacent electrodes extend towards the edge of the biosensor, at least one electrode does not extend to the edge of the biosensor; or on the path that an electrode and a non-electrode conductive block adjacent thereto extend towards the edge of the biosensor, at least one of the electrode and the non-electrode conductive block does not extend to the edge of the biosensor.

9. The method according to claim 7, wherein a non-electrode conductive block is disposed between at least one of two adjacent electrodes and the edge of the biosensor.

10. A method of avoiding a short circuit of electrodes of a biosensor, wherein on the path that two adjacent electrodes extend towards the edge of the biosensor, at least one electrode does not extend to the edge of the biosensor; or on the path that an electrode and a non-electrode conductive block adjacent thereto extend towards the edge of the biosensor, at least one of the electrode and the non-electrode conductive block does not extend to the edge of the biosensor; and a non-electrode conductive block is disposed between at least one of two adjacent electrodes and the edge of the biosensor.
